# EUROPEAN PATENT APPLICATION

(11) **EP 2 545 908 A1**
(43) Date of publication of application: **16.01.2013**
(21) Application number: 11173435.6
(22) Date of filing: 11.07.2011
(51) Int. Cl.: A61K 9/50, A61K 9/51, A61K 9/10, A61K 49/22

(54) **Medium for microbubbles or microparticles and preparation thereof**

(71) Applicant: RWTH Aachen, 52062 Aachen (DE)
(72) Inventor: Hasbach, Michael, 16341 Panketal (DE); Hauff, Peter, Dr., 10439 Berlin (DE); Kießling, Fabian Prof., 52074 Aachen (DE)
(74) Representative: Kröncke, Rolf

(57) **Abstract**

The present invention relates in a first aspect to a suspension of microbubbles or microparticles in an aqueous liquid medium. Said suspension is usable for diagnostic, therapeutic and analytic purposes comprising microbubbles and/or microparticles and/or nanoparticles whereby said liquid medium contains dissolved shelf material of said microbubbles, microparticles or nanoparticles in a concentration avoiding the dissolution of said microparticles, microbubbles or nanoparticles or avoiding the loss of acoustic properties or other properties of said microbubbles, microparticles or nanoparticles. In another aspect, the present invention relates to a method for preparing a suspension containing microbubbles or microparticles in an aqueous liquid medium to achieve improved shelf life. Moreover, an aqueous liquid storage and handling solution for microbubbles or microparticles as well as for nano-particles is provided. Finally, pharmaceutical compositions containing the same are disclosed.

## Description

The present invention relates in a first aspect to a suspension of microbubbles or microparticles as well as nanosized bubbles and particles in an aqueous liquid medium. Said suspension is usable for diagnostic, analytic and therapeutic purposes comprising microbubbles and/or microparticles as well as nanosized bubbles and particles whereby said liquid medium contains dissolved shell material of said microbubbles or microparticles as well as nanosized bubbles and particles in a concentration avoiding the dissolution of said microparticles or microbubbles as well as nanosized bubbles and particles or avoiding the loss of acoustic properties or other properties of said microbubbles or microparticles as well as nanosized bubbles and particles. In another aspect, the present invention relates to a method for preparing a suspension containing microbubbles or microparticles as well as nanosized bubbles and particles in an aqueous liquid medium having improved shelf life. Moreover, an aqueous liquid storage and handling solution for microbubbles or microparticles as well as for nanosized bubbles and particles is provided. Finally, pharmaceutical compositions containing the same are disclosed.

### Background

Microbubbles or microparticles in micro- or nanometer size are used in various applications. In particular, for the therapeutic and/or diagnostic purposes the use of nano- or micrometer-sized bubbles or particles gains high attention and wide application. For example, the *in vivo* imaging of processes at the cellular and molecular level is an important part in better understanding physiological and pathophysiological procedures at the molecular level and offers new opportunities for diagnosing and treating oncological, cardiovascular and other diseases. Various techniques have been described allowing *in vivo* imaging. Examples thereof include positron emission tomography (PET) and SPECT (single photon emission computed tomography) representing well established molecular imaging technologies in clinical diagnostics by the detection of radioactively labelled probes. However, these techniques still have a low spatial resolution. For this reason, PET and SPECT are often combined with other imaging procedures, like CT or MRI. Thus, it is possible to combine anatomical with cellular or molecular or metabolic images providing high resolution imaging procedures. The suitability of molecular imaging has already been established at the clinical or research levels for other procedures, such as optical, magnetic and ultrasound imaging technologies. For ultrasound imaging technologies, ultrasound contrast agents (USCAs) with specific acoustic properties have been described. These USCAs provide a contrast when used with ultrasonic waves. In this connection, contrast generally defines the intensity ratio of two neighbouring pixels, shown in the image replication. That is, the ultrasound imaging technologies rely on measuring the density and imaging the differences in density of structures of e.g., particular body compartments or tissues. To enhance the differences in density, the USCA have been developed.

Gas bubbles (microbubbles) have proven to be particularly advantages as USCA since gases process low acoustic impedance (i.e. low density) when compared with a surrounding tissue. The large differences in acoustic impedance between gases and tissue, the so called impedance jump, lead to an intensified reflection of acoustic waves at their interface.

Thus, the use of microbubbles and microparticles as USCA in diagnosis and therapy is generally accepted. That is, these types of USCA have been used widely in diagnostic applications in medical research and clinical practice. Generally, according to the current point of view, the various USCA formulations based on microbubbles or microparticles can be divided into six categories based on their morphology, how they function and their indication. These six categories are shown in table 1 below.

**Table 1: Organization of the USCA formulation into 6 categories.**

| **Category** | **Formulation** | **Characteristics** |
|---|---|---|
| 1 | Free gas bubble | Dissolves rapidly in blood and do not pass the capillary bed of the lung (right-heart CA) |
| 2 | Encapsulated gas bubble with a soft elastic lipid or protein shell (soft shell) | Stabilized gas bubble, which can pass the capillary bed of the lung (CA for the entire cardiovascular system) |
| 3 | Encapsulated soft shell gas bubble, whereby slightly soluble gases are used here (e.g. Perfluoro gases) | Gases with longer acoustic life span in the cardiovascular-system |
| 4 | Encapsulated gas bubble with a stable shell consisting of biodegradable polymers such as polylactide or cyanoacrylate (hard shell) | Gas bubbles with longer half live and specific acoustic characteristics such as Stimulated Acoustic Emission (SAE) |
| 5 | Encapsulated gas bubble with target-specific ligands on the shell surface | USCA for molecular imaging |
| 6 | Gas bubbles as carriers of therapeutic substances | Gas bubbles as drug delivery system (DDS) |

Typically, the USCAs are minuscule microbubbles with a size in the vicinity of 1 to 5 µm, which are stabilised with a thin shell. However, USCA including microbubbles and microparticles in the nano-type range have been described as well. In addition, various forms of USCAs are known in the art including the referenced soft and hard shell types, nano-sized and stabilised perfluorcarbon liquid drops which enlarge after ultrasound treatment only, thus acquiring their ultrasound contrast activity.

Further, ultrasound contrast agent includes microbubbles and microparticles having a specific type of electrostatic surface charge, having a specific chemical composition of the shell or consisting of different types of gas such as e.g., air, nitrogen, oxygen, carbon dioxide, inert gases, alkane, alkene, alkyne or perfluoro-carbon-hydrogens or mixture of different type of gases.

For example, it is described in EP 0 921 807 that gas-mixtures containing hydrogen gas may be administered in liposomes, or gas-filled microparticles to patients. Administrations of liquids in microbubbles or microparticles are described in various documents, e.g. US 2003/0157023.

Microbubbles include aqueous suspensions in which the bubbles of gas are bounded at the gas/liquid interface by thin envelope involving a stabilised amphiphilic material disposed at the gas to liquid interface. Microbubbles suspensions are typically prepared by contacting powdered amphiphilic materials, e.g. freeze-dried preformed liposomes or freeze-dried or spray-dried phospholipids solutions, with air or other gas and then with an aqueous carrier, while agitating to generate a microbubble suspension which can then be administered, preferably shortly after its preparation. Examples of aqueous suspension of gas microbubbles and preparations thereof are disclosed for instance in US 5,271,928 or US 5,445,813. For example, gas-filled microparticles include suspensions in which the bubbles of gas are surrounded by a solid material envelope of a lipid or of natural or synthetic polymers. Typically, gas-filled microparticles have a nucleus-shell structure whereby the thickness of the shell or envelope may vary from a few nanometres to a few micrometers. Examples of gas-filled microparticles and of the preparation thereof are disclosed for instance in US 5,711,933 and US 6,333,021.

Microbubbles and microparticles, like gas microbubbles and gas-filled microparticles, are used both, in diagnostic and therapeutic approaches. That is, microbubbles and microparticles may represent potential drug carriers allowing target directed delivery of the drugs. This is particularly true for non-functionalised and functionalised microbubbles and microparticles. For example, functionalised gas-filled microparticles are described in US 2003/0157023. Herein, the gas-filled microparticles are functionalised by e.g. hydrolysing of a polyalkylcyanoacrylate shell. These gas-filled microparticles are particularly useful in ultrasound diagnosis.

Indeed, microbubbles and microparticles are used as contrast agents - in particular ultrasound contrast agents - since many years. A review about the currently available microbubbles and microparticles for use as ultrasound contrast agents is provided in Kiessling, F., et.al., 2009 Curr Med Chem, 16:627-42. Therein, an overview is provided about the development and state of the art of ultrasound contrast agents based on microparticles including soft-shell microbubbles and hard-shell microparticles. Moreover, said document identifies advantages and disadvantages of the different types of microparticles, in particular with respect to ultrasound diagnosis including stability and sensitivity.

Using functionalised microbubbles or microparticles provide the possibility of specifically binding or conjugating molecules or substances. Said molecules may be compounds allowing targeting of the microparticles influencing physical and mechanical properties of the microparticles or influencing the metabolism in the subject. That is, functionalisation allows altering the degradation and the metabolisation properties of the microbubbles and microparticles. Further, using functionalised microbubbles or microparticles modified by comprising a molecule or moiety conjugated or bound via the functionalised group allow targeting of said microbubbles or microparticles. USCA targeting of appropriate targets may be achieved by passive targeting or active targeting. Passive targeting is a rather non-specific target enhancement by microbubbles or microparticles, for example based on size, chemical properties and electrical charge of the shell as well as the type of encapsulated gas, the route of injection and by physiological processes of the immune system. In particular, the following principle mechanisms have been described for passive targeting: phagocytosis, interaction with the cell membranes and lymph flow transport.

Active targeting requires special molecular structures allowing targeting to specific binding partners. Of course, this requires knowledge of the molecular targets within the living organism in certain physiological and pathophysiological situations and the possibility to select said special molecular structures allowing binding to the specific binding partners for later imaging. USCAs allowing active targeting are composed of a shell-stabilised gas microbubble and a targeting moiety which may optionally linked by a linker moiety to the shell. The targeting moiety is connected with the surface of functionalised microbubbles or functionalised microparticles and, typically, the targeting molecular structures are ligands selected from antibodies, peptides, polysaccharides, lectines or aptamers. The coupling or linkage of the targeting moiety to the signalling moiety, namely, the functionalised microbubbles or functionalised microparticles may be achieved by direct or indirect connection. Direct connection means covalent coupling optionally via a linker molecule while indirect connection means coupling e.g. via the biotin-streptavidin system wherein no covalent linkage is present. However, for clinical application, the targeting moiety should be coupled preferably by covalent binding to the signalling moiety.

Molecular imaging with targeted ultrasound contrast agents of microbubbles or microparticles is possible. For example, molecular imaging of tumor angiogenesis using VEGFR-2 or lymph nodes using L-selectin are described (Hauff P. et al., Handbook of Exp. Pharmacology 2008 (185), 223-245). Hence, functionalised microbubbles or gas-filled microparticles, e.g. cyanoacrylate microbubbles, conjugated to ligands including antibodies represent a useful tool for enhancing specificity and sensitivity of the ultrasound diagnosis.

In US 2004/0180004 a stable microbubble suspension as enhancement agents for ultrasound echography and dry formulations thereof are disclosed. It is identified that the injectable suspension of microbubbles in an aqueous carrier liquid comprises at least 10⁷ microbubbles/ml and amphiphilic compounds at least one of which is a phospholipid stabiliser of the microbubbles against collapse, characterised in that the concentration of the phospholipids in the carrier is below 0.01 % by weight while being equal to or above that at which the phospholipid molecules are present solely at the gas microbubble-liquid interface. The patent application identifies that suspension with high microbubble concentrations having relatively high stability and long storage capacity may be prepared even if the concentration of the phospholipid as surfactants are kept well below the levels known in the art identifying that low amounts of amphiphiles are sufficient to stabilise microbubbles.

It is generally desired to apply the microbubbles or microparticles in concentrations as low as possible. In addition, it is highly desired to provide stable compositions containing microbubbles or microparticles ready for use, thus, avoiding unnecessary work e.g. diluting or reconstitution of respective suspensions before application to an individual.

However, according to the art, the microbubbles or microparticles commercially available (e,g, Definity®, Optison^{™} or SonoVue), when used as ultrasound contrast agents, are administered in amounts of approx. 2.5 x 10⁸ up to more than 10¹⁰ microbubbles or microparticles per patient to allow ultrasound diagnosis for identification, localisation and characterisation of diseases, disorders or conditions. These doses correspond with approx. 5mg up to 170mg solid matter per patient depending on the type of USCA. Therefore, the minimum recommended doses of all commercially available USCA are far above microdosing, hence, several adverse reactions are listed in the respective package leaflet.

Generally, microdosing is a technique for studying the behaviour of drugs in humans by the administration of doses so low that they are unlikely to produce whole-body effects, but high enough to allow monitoring the event to be studied. This allows evaluating the pharmacokinetics of new drug candidates with almost no risk of side effects. The dose of a new drug for human volunteers is typically about 100 times lower than the proposed e.g. therapeutic or diagnostic dosage (from approx. 1 to 100 micrograms per volunteer but not above).

As mentioned before, all commercially available USCA are far above microdosing and have therefore the potential for adverse reactions in human. Therefore, there is a need to provide ultrasound contrast agents allowing microdosing to avoid adverse reactions while maintaining sufficient sensitivity. Furthermore, extensive work and moneys, required to obtain approval of new microparticles or microbubbles as medicinal products by the respective authorities (e.g., EMEA, FDA), can be dramatically reduced by USCA microdosing.

Thus, the object of the present invention is to provide suspensions of non-functionalised or functionalised microbubbles or non-functionalised or functionalised microparticles in an aqueous liquid medium being usable for diagnostic and therapeutic purposes whereby said suspensions have improved stability and long storage capacity. That is, the suspensions of the present invention are characterised in showing less collapsing or degradation of the microbubbles or microparticles during storage. In particular, the suspension is characterised in that microparticles or microbubbles can be stored for microdosing in suitable concentrations of said microbubbles or microparticles.

### Summary of the present invention

Hence, in a first aspect, the present invention relates to a suspension of non-functionalised or functionalised micro- or nanobubbles or non-functionalised or functionalised micro- or nanoparticles in an aqueous liquid medium, usable for diagnostic, analytic and therapeutic purposes. The aqueous liquid medium is characterised in containing dissolved shell material of said micro- or nanobubbles or micro- or nanoparticles in a concentration avoiding substantive dissolution or the loss of acoustic properties or other properties of the micro- or nanobubbles or micro- or nanoparticles suspended therein.

The present inventors recognised that dissolved shell material present in the solvent, e.g. the aqueous liquid medium, avoid substantial disintegration or decomposition of the micro- or nanobubbles or micro- or nanoparticles.

That is, the solvent contains the dissolved shell material present in form of monomers and/or polymers for preventing degradation or decomposition of the bubbles or particles. In particular, an equilibrium between dissolved shell material and shell material present in the bubbles or particles allow to stabilise the bubbles and particles in the suspension, accordingly. Thus, it is possible to provide ready-to-use solutions showing prolonged storage and handling characteristics. Further, the stability of the bubbles and particles is improved.

Moreover, it is possible to store and handle micro- or nanobubbles or micro- or nanoparticles in low concentration in a liquid medium without the problem of decomposition or degradation of said bubbles or particles. This allows e.g. the preparation of singles dosages even for small laboratory animals such as mice. Moreover, the said stabilization medium offers the possibility of selective and reproducible analytics of single microbubbles, microparticles or nanoparticles, with a big benefit / advantage regarding of e.g. the process and quality monitoring during or after the production of microbubbles, microparticles or nanobubbles or nanoparticles.
Furthermore, the said stabilization medium offers the possibility to work only with low concentrations of microbubbles, microparticles or nanoparticles during different steps of processes such as e.g. functionalizing or labelling with target specific molecules.

In a preferred embodiment, the suspension of micro- or nanobubbles or micro-or nanoparticles in an aqueous liquid medium according to the present invention contains said micro- or nanobubbles or micro- or nanoparticles in a concentration of below 5x10⁷ micro- or nanobubbles or micro- or nanoparticles/ml, preferably the concentration is below 5x10⁵ micro- or nanobubbles or micro- or nanoparticles/ml.

Moreover, the suspension of micro- or nanobubbles or micro- or nanoparticles in an aqueous liquid medium according to the present invention is a ready-to-use suspension. That is, the suspension is in a form which allows direct application to an individual. Furthermore, it is preferred that the suspension is sterile.

It is preferred that the concentration of dissolved shell material and micro- or nanobubbles or micro- or nanoparticles is adjusted in a way that no decomposition or degradation of said bubbles or particles occur. The skilled person is well aware of suitable methods for determining the same. That is, it is preferred the shell material present in the medium is in equilibrium with the shell material present in the bubbles or particles. Furthermore, it is preferred that the total amount of shell material present in the suspension according to the present invention allows to administer to an individual the suspension in a concentration of below 143 µg/kg body weight, preferably below 1.43 µg/kg body weight.

In another aspect, the present invention relates to a method for preparing a suspension according to the present invention comprising the step of preparing the micro- or nanobubbles or micro- or nanoparticles in a reaction medium, optionally, in the presence of a surfactant. The reaction medium containing dissolved components of the shell forming material of said bubbles or particles is diluted further in a way to bring the reaction medium in equilibrium with the micro- or nanobubbles or micro- or nanoparticles, thus, avoiding the dissolution or the loss of acoustic properties or other properties of said bubbles or particles. Alternatively, the reaction medium may be prepared by freshly dissolving the components of the shell material in an aqueous liquid medium in a concentration sufficient to bring the dissolved shell forming material in equilibrium with the micro- or nanobubbles or micro- or nanoparticles suspended therein, thus, avoiding dissolution of said micro- or nanobubbles or micro- or nanoparticles.

Further, an aqueous liquid storage and handling solution for micro- or nanobubbles or micro- or nanoparticles is provided. Finally, the present invention relates to pharmaceutical compositions containing the suspension of the present invention and its use for diagnostic or therapeutic purposes. The suspension may be combined with known diagnostic methods, like PET or SPECT. Thus, the suspension is particular useful allowing diagnosis with diagnostic methods including PET and SPECT, optionally the micro- or nanobubbles or micro- or nanocapsules may be coupled or conjugated with a suitable diagnostic marker or label.

### Brief description of the drawings

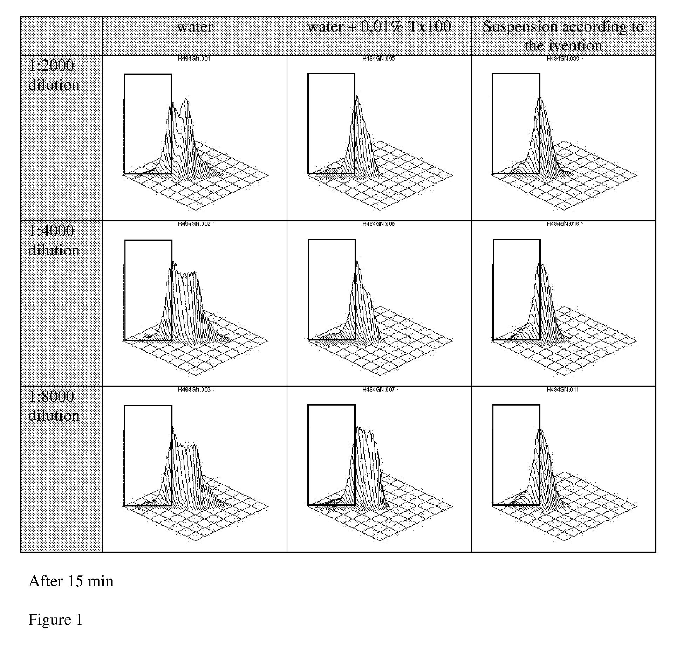

In figure 1, the distribution of microparticles is shown in various dilutions. Shown are flow cytometry analyses with samples diluted in water, water with surfactant and particles dissolved in an aqueous liquid storage and handling solution according to the present invention, namely a suspension of microparticles or microbubbles according to the present invention. Figure 1 shows the results of the 15 minutes incubation.

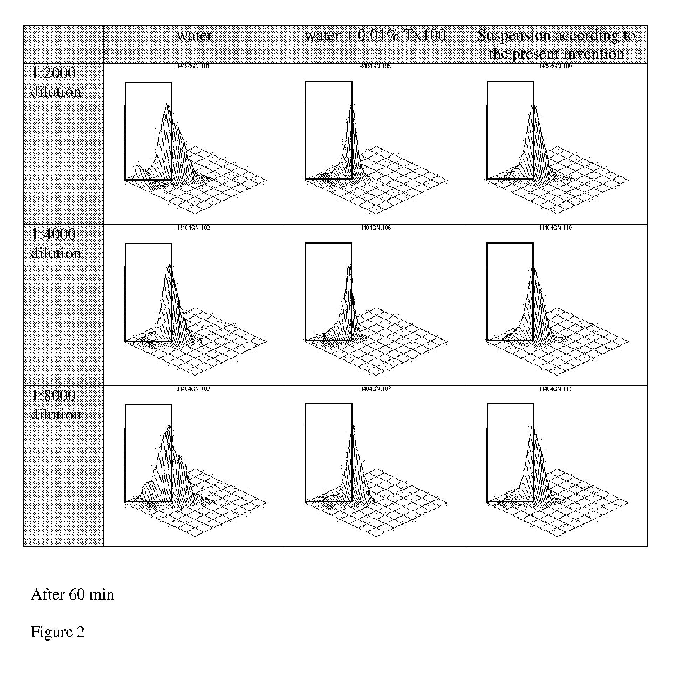

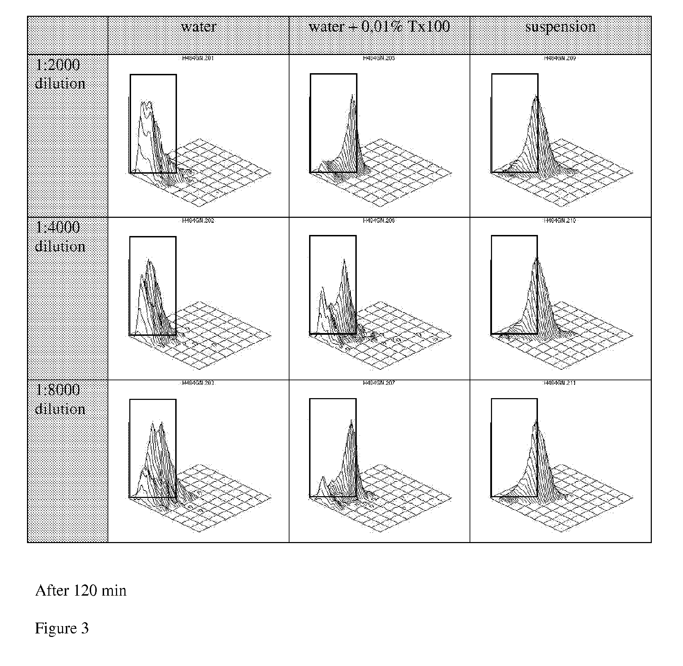

In figure 2, the results are shown after 60 minutes incubation of microparticles in respective solvents. Figure 3 shows the distribution of microparticles after 120 minutes incubation.

It is demonstrated in the figures that the microparticles are stable in the suspension according to the present invention only.

### Detailed description of the present invention

The present inventors recognised that it is possible to provide suspension containing micro- or nanobubbles or micro- or nanoparticles even in low concentrations of below 10⁸ bubbles/ml, like 10⁷, and, preferably, in concentrations below 10⁶ bubbles/ml with high stability and long storage capacity when using an aqueous liquid medium as a solvent containing shell material of said micro- or nanobubbles or micro- or nanoparticles in a concentration avoiding the dissolution or the loss of acoustic properties or other properties of said micro- or nanobubbles or micro- or nanoparticles.

Unless otherwise indicated, the term "bubbles" and the term "particles" include microbubbles and nanobubbles as well as microparticles and nanoparticles as defined herein. Said bubbles and particles may be in a non-functionalised or functionalised form.

As used herein, the term "dissolved shell material" refers to components forming the shell of the micro- or nanobubbles or micro- or nanoparticles. The components may be in form of unreacted monomers or partially polymerisated monomers, e.g in form of oligomers, or polymers. In addition, the dissolved shell material refers to components which may be in form of fragments of the bubbles or particles in form of deaggregated polymers etc.

In addition, the dissolved shell material may comprise or consist of other components, either monomers or polymers or fragments thereof, not forming the shell of the bubbles or particles, respectively. That is, the shell material may be a propylene-polymer while the components present in the solution may be an ethylene-polymer or fragments thereof or may be a mixture of propylene- and ethylene-polymer or fragments thereof.

In addition, it is preferred that the shell material as well as the components dissolved in the solution are biodegradable materials and components.

Further, the term "in a concentration avoiding the dissolution" as used herein refers to the concentration of the components of the shell material dissolved in the liquid medium. The concentration is adjusted to be substantially in equilibrium with the microbubbles, microparticles or nanobubbles or -particles suspended in said medium.

The term "loss of acoustic properties or other properties" refers to decreased backscatter intensity of the ultrasound pulse and less non linear reflections as well as to significant alteration of blood circulation time.

The term "loss of functionality" includes all desired functions / properties of respective microbubbles, microparticles or nanobubbles or -particles

In the context of the present invention, the following terms are used:
- Microparticles:: A generic term for all particles and capsules containing gas or gas-forming substances (e.g. liquids) or other substances as cores which are stabilized by a so called hard shell and measuring between 1 µm and 1000 µm, regardless of their structural design including being of solid structure.
- Microbubbles:: A generic term for all a) bubbles, in particular, ultrasound contrast agents, consisting of gas or gas-forming substances (e.g. liquids) containing other substances or not which are non-stabilized or b) bubbles, in particular, ultrasound contrast agents, containing gas, gas forming substances or other substances as core which are stabilized by a so called soft shell, both measuring between 1000 µm and 1000 µm, regardless of their structural design.
- Nanoparticles:: A generic term for all particles and capsules containing gas or gas-forming substances (e.g. liquids) or other substances as cores which are stabilized by a so called hard shell and measuring between 1 nm and up to 999 nm, regardless of their structural design. They can also be of solid structure.
- Nanobubbles:: A generic term for all a) bubbles, in particular, ultrasound contrast agents, consisting of gas or gas-forming substances (e.g. liquids) containing other substances or not which are non-stabilized or b) bubbles, in particular, ultrasound contrast agents, containing gas, gas forming substances or other substances as core which are stabilized by a so called soft shell, both measuring between 1 nm and up to 999 nm, regardless of their structural design.

It is preferred that the micro- or nanoparticles or micro- or nanobubbles according to the present invention are gas-filled microparticles or nanoparticles (GFMP) or gas-filled microbubbles or nanobubbles. Of course, it is also possible that the micro- or nanoparticles have a solid structure.

The term "alkylcyanoacrylate" refers to an alkylester of cyanoacrylic acids. Polyalkylcyanoacrylate are polymers made of one or more alkylcyanoacrylates essentially without free acid and alcohol groups.

With the term "functionalised micro- or nanobubbles" or "fuctionalised micro- or nanoparticles" is meant bubbles or particles having functional groups. Basically, functionalisation refers to the presence of functionalised monomers and copolymers formed from said monomer whereby the functional groups are free for later reaction with other molecules or moieties conjugating the same with the bubbles or particles. A functional group refers to a molecule group that contains at least one polar, reactive atomic compound with an X-H-group of atoms, with x = O, S and N. Functional monomers are monomers containing a functional group whereby said functional group may be a free functional group or a protected functional group. Protected functional group (non-functional group) refers to a functional group which is not available for reaction due to the presence of protective groups known in the art. A non-functional group can be converted into a functional group by cleavage of their protective group.

The term "microdosing" refers to a dosing of < 100 µg/70 kg body weight, corresponding to 1.43 µg/kg body weight. In this connection, the amount of components is given for both, the components dissolved in the suspension or solution and the components forming the bubbles or particles.

The term "high dosage" refers to a dosage far above microdosing and is typically in the range between 5mg and 170mg per human for commercially available USCA.

The term "targeting moiety" refers to a compound or moiety allowing to specifically bind the bubbles or particles, in particular, the functionalised bubbles or functionalised particles, to cells, their epitopes or receptors (predominantly vascular or inflammation cells) or components of the interstitial matrix (such as fibronectin or collagen). This can be e.g. an antibody, a fragment of an antibody, a peptide, an aptamer, a lectin, nucleic acid or similar molecules. The term "targeting moiety" includes diagnostic marker molecules and therapeutic molecules.

The term "diagnostic marker molecule" refers to a compound or moiety allowing diagnosis of a disease, disorder or condition.

The term "therapeutic molecule" refers to a compound or moiety allowing to treat diseases by specifically binding to a structure in vivo. This can be e.g. an antibody, a fragment of an antibody, a peptide, an aptamer, a lectin, nucleic acids or similar molecules. The therapeutic molecule may also be used for diagnosis in lower dosages. Combined diagnostic and therapeutic compounds (which can also include drug loaded micro- or nanobubbles or micro- or nanoparticles) are called diagnostic agents.

It was recognised that microdosing of ultrasound contrast agent based on non-functionalised or functionalised bubbles or non- functionalised or functionalised particles minimises the risk of adverse side effects and allergic reactions as well as enables reducing the costs for approval for medicinal products while maintaining the desired sensitivity of diagnosis.

It is particularly preferred that the non- functionalised or functionalised bubbles or non- functionalised or functionalised particles are administered in a dosage of functionalised bubbles or gas-filled particles of < 100 µg/70 kg body weight.

Said functionalised bubbles or particles are preferably capsules comprising functionalised polyalkylcyanoacrylates or polylactic acids. Functionalised polyalcylcyanoacrylates are described e.g. in US 2003/0157023 A1 which is incorporated herein fully by reference. That is, the functionalised particles or functionalised bubbles comprise functionalised polyalkylcyanoacrylates optionally in combination with non-functionalised polyalkylcyanoacrylates. In particular, the functionalised particles or functionalised bubbles are gas-filled particles or functionalised bubbles containing functionalised polyalkylcyanoacrylates. The functionalised polyalkylcyanoacrylate can be produced by a copolymerization of one or more alkylcyanoacrylates preferably butyl-, ethyl- and/or isopropylcyanoacrylate with a functional monomer, preferably, cyanoacrylic acid, and/or by a partial side-chain hydrolysis of a polyalkylcyanoacrylate, preferably polybutyl-, polyethyl- and/or polyisopropylcyanoacrylate. Suitable functional monomers are:

Cyanoacrylic acid (H₂C=C(CN)-CO-OH), methacrylic acid (H₂C=C(CH₃)-CO-OH), methylenemalonic acid (H₂C=C(CO-OH)₂) and α-cyanosorbic acid (H₃C-CH=CH-CH=C(CN)-CO-OH) and their derivatives with the general formulas:
H₂C=C(CN)-CO-X-Z (cyanoacrylic acid derivatives),
H₂C=C(CH₃)-CO-X-Z (methacrylic acid derivatives),
H₂C=C(CO-X'-Z')₂ (methylenemalonic acid derivatives) and
H₃C-CH=CH-CH=C(CN) -CO-X-Z (α-cyanosorbic acid derivatives) with
X = -O-, -NH- or -NR¹- and
Z = -H, -R²-NH₂, -R²-NH-R¹, -R²-SH, R²-OH, R²-HC(NH₂)-R¹ 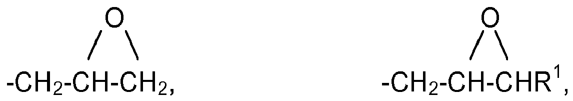 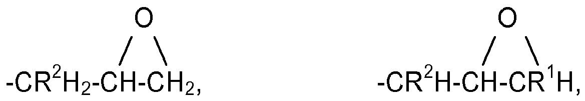
whereby R¹ = linear or branched alkyl radical and R² = linear or branched alkylene radical with respectively 1 up to 20 carbon atoms, and whereby both X' and Z', in each case independently of one another, have the meaning that is indicated for X and Z.

Substituted styrenes (Y-C₆H₄-CH=CH₂) or methylstyrenes (Y-C₆H₄-C(CH₃)=CH₂) with Y = -NH₂, -NR¹H, -OH, -SH, -R²-NH², -R²-NH-R<1>, -R²-SH, R²-OH, -R²-HC (NH₂)-R¹, whereby R¹ = linear or branched alkyl radical and R² = linear or branched alkylene radical with respectively 1 up to 20 carbon atoms.

Alternative polymeric substances or their monomeric precursors may be a) homopolymers such as polyethylene, polypropylene, polyvinylchloride and polyamide; b) copolymers such as polyester, polyurethane and some polyamide; c) natural biopolymers such as polysaccharides, proteins and peptides, and polynucleotide; d) bio-based polymers such as polylactids or polyhydroxybutyrats; e) synthetic biopolymers such as polyesters, polyvinyl-alcohols, polybutylenadipatterephthalat, polybutylensuccinat, polycaprolactone, polyglycolids or f) synthetic polymers such as polyethylene, polystyrole or polyvinylchloride. Furthermore, silane containing compounds or denaturized proteins are also included.

It is preferred that the functionalised bubbles or functionalised particles are conjugated with a targeting moiety (e.g. a diagnostic marker molecule or a therapeutic molecule) which is conjugated to the functionalised bubbles or functionalised particles by coupling the same via the functionalised functional group. For example, the coupling of the targeting moiety, like a diagnostic marker molecule or a therapeutic molecule may be affected via carboxylic acid groups as described in Kiessling F., et. al., 2009, Current Medicinial Chemistry, 16(5), 627-642.

In another embodiment, the functionalised bubbles or functionalised particles are conjugated with streptavidin or avidin. Said streptavidin or avidin conjugated functionalised bubbles or functionalised particles allows to non-covalently attach further molecules, including diagnostic marker molecules, therapeutic molecules or targeting molecules having a biotin moiety. Hence, a non-covalent binding system of functionalised bubbles or functionalised particles is provided.

That is, the present invention relates to a suspension of non-functionalised or functionalised bubbles or non-functionalised or functionalised particles in an aqueous liquid medium, usable for diagnostic and therapeutic purposes comprising said bubbles or particles characterised in that the liquid medium contains dissolved shell material of said bubbles or particles in a concentration avoiding the dissolution or the loss of acoustic properties or other properties of said bubbles or particles.

It is preferred that the concentration of the bubbles or particles in the suspension according to the preset invention is below 1x10¹⁰ bubbles or particles/ml, preferably below 1x10⁹ bubbles/ml. It is particularly preferred that the concentration of said bubbles or particles is below 5x10⁸/ml like below 1x10⁸/ml. It is in particular preferred that the suspension of the present invention allows microdosing of the bubbles or particles with further dilution before use. That is, it is particularly referred to provide a valid use suspension of bubbles or particles in an aqueous liquid medium wherein the concentration of said bubbles or particles is below 5x10⁷/ml, preferably below 5x10⁶ bubbles or particles/ml. In a most preferred embodiment, the concentration of bubbles or particles in the suspension according to the present invention is below 5x10⁵ bubbles or particles/ml, like below 1x10⁵ bubbles or particles/ml.

Hence, the suspension of bubbles or particles in an aqueous liquid medium according to the present invention allows to administer the same in concentrations below 143 µg/kg body weight, preferably below 14.3 µg/kg body weight of the total amount of the shell forming material including the dissolved shell forming material and the shell forming material in form of the particles or bubbles.

It is preferred that the total concentration of the shell forming material is below 1.43 µg/kg body weight, roughly corresponding to the administration of particles or bubbles in a concentration of in between 1x10⁵ bubbles/ml and 5x10⁵ bubbles/ml.

The suspension of the present invention may contain additional low amounts of surfactant, like non-ionic surfactant. That is, small or very small amounts of suitable surfactants may stabilise the bubbles and particles as described in the art.

The dissolved substances of the shell material present in the aqueous liquid medium according to the present invention may be in the form of polymers or polymeric aggregates as well as in form of monomers or oligomers. It is preferred that the shell material dissolved in the aqueous liquid medium is identical with the polymer and/or monomer composition of the bubbles or the particles.

In another preferred embodiment, the suspension of bubbles or particles in an aqueous liquid medium according to the present invention is in form of a ready-to-use solution. Thus, no prior dilution or resuspension or reconstitution of frozen or lyophilised bubbles or particles is required. Hence, the suspension of bubbles or particles in the aqueous liquid medium allows easy-to-use application in practice. In particular, the present invention allows providing ready-to-use suspensions containing the bubbles or particles in low concentrations of below 10⁷ bubbles or particles/ml, preferably below 10⁶ bubbles or particles/ml.

It is apparent that preformed bubble suspension, e.g. bubble or particle contrast agent systems, desirably exhibit good storage stability, for example, in order to permit manufacture at a central location and distribution to and storage at hospitals etc. prior to use. This is particularly true for suspensions allowing microdosing of bubbles or particles.

In addition, the aqueous liquid storage solution for bubbles or particles as described herein are useful in analytic approaches, e.g. analytic characterisation of single bubbles or particles. This will be helpful in the field of process and quality control when preparing the bubbles or particles, in particular, with respect to pharmaceutical products in preclinical and clinical application.

Moreover, the storage and handling solution according to the present invention allows to perform downstream processes with the bubbles or particles. Said downstream processes include washing and labelling or conjugating otherwise the bubbles or particles with diagnostic or therapeutic components, like marker, label, diagnostic marker molecules, therapeutic molecules or targeting molecules. For example, conjugation of VEGFR-2 receptor to functionalised bubbles or functionalised particles are described. In addition, other conjugated bubbles and particles conjugated with antibodies etc. are known in the art. With the suspension and aqueous storage solution according to the present invention, it is possible to conduct the downstream processes with low concentrations of bubbles and particles as well as with a lower concentration of the molecules to be labelled or conjugated. Hence, the present invention allows to safe costs and time, accordingly.

Moreover, the ready-to-use solution according to the present invention can be stored over a longer time with lower concentrations of bubbles or particles. Thus, economic advantages are given as well as allow manufacturing at a central location and distribution to a storage at hospitals prior to use without additional work at the hospital.

The present invention relates in another aspect to a method for preparing a suspension of non-functionalised or functionalised bubbles or non-functionalised or functionalised particles in an aqueous liquid medium having improved shelf life comprising the step of
a) providing bubbles and/or particles,
b) providing an aqueous liquid medium containing dissolved shell material of the bubbles or particles according to step a) in a concentration avoiding the dissolution or the loss of acoustic properties or other properties of said bubbles or particles.
c) suspending the bubbles and/or particles of step a) in the aqueous liquid medium of step b) to provide a suspension of non-functionalised or functionalised bubbles or non-functionalised or functionalised particles in an aqueous liquid medium having improved shelf life.

In one embodiment of the method according to the present invention the bubbles or particles are prepared in a reaction medium containing monomers for forming said bubbles and/or particles and, optionally, a surfactant. Optionally separating the prepared bubbles or particles from the reaction medium and diluting the reaction medium containing dissolved components of the shell forming material of said bubbles or particles to a concentration being in equilibrium with the bubbles or particles properties of said bubbles or particles. In a further step, the diluted reaction medium and the separated bubbles or particles are reunified by suspending said bubbles or particles in said diluted reaction medium.

In a preferred embodiment, the method for preparing a suspension of non-functionalized or functionalized bubbles or non-functionalized or functionalized particles in an aqueous liquid medium having improved shelf life comprises the steps of
a) preparing particles and/or bubbles in a reaction medium containing monomers and, optionally, a surfactant;
b) separating the bubbles or particles obtained in step a), e. g. by floating;
c) optionally, diluting the remaining reaction medium with an aqueous solution, preferably water, and stirring said reaction medium;
d) allowing the reaction medium to stay for a predetermined time for obtaining a reaction medium containing monomeric or polymeric components.
e) mixing bubbles or particles, e.g. obtained in step b), with the reaction medium obtained in step d) to obtain a suspension of bubbles or particles in an aqueous liquid medium.

It is preferred that the aqueous liquid medium, like the reaction medium, is a medium being saturated with the monomeric or polymeric components forming the bubbles or particles. That is, depending on the concentration of the bubbles or particles present in the aqueous liquid medium, the concentration of the dissolved components is adjusted in a way that the dissolved components are in equilibrium with the bubbles or particles. Thus, no degradation or decomposition of the bubbles or particles occurs. In another aspect, the present invention relates to an aqueous liquid storage and handling solution for bubbles or particles, optionally comprising a surfactant, characterised in that the medium contains dissolved components of the shell material of said bubbles or particles to be stored therein in a concentration sufficient to avoid the dissolution or the loss of acoustic properties or the properties of the mentioned bubbles or particles.

Said aqueous liquid storage solution allows to store suspended bubbles or particles and permit to manufacture suspended bubbles or particles e.g. at a central location while distribution to a storage at hospitals prior to use is possible.

That is, the aqueous liquid storage and handling solution according to the present invention is particularly useful for preparation of ready-to-use solutions of suspended particles or bubbles.

In another aspect, the present invention relates to a pharmaceutical composition having extended shelf life containing non-functionalised or functionalised bubbles or non-functionalised or functionalised particles in an aqueous liquid medium for diagnostic and therapeutic purposes in a suspension according to the present invention.

It is particular preferred that the pharmaceutical composition according to the present invention is a preparation wherein the non-functionalised or functionalised bubbles or non-functionalised or functionalised particles are present in a concentration of below 5x10⁸ bubbles or particles/ml, preferably 5x10⁷, like 5x10⁶, in particular, below 5x10⁵ bubbles or particles/ml.

It is particularly preferred that the pharmaceutical composition according to the present invention is in a form of a ready-to-use solution or in a form of a presuspension having an extended shelf life. The pharmaceutical composition includes compositions for diagnostic and therapeutic purposes. The pharmaceutical or diagnostic composition according to the present invention may contain additional components typically present in pharmaceutical or diagnostic compositions.

Administration of the pharmaceutical or diagnostic composition according to the present invention may be effected in various ways depending on its specific formulation. Examples of suitable administration are, for instance, intravasculary, intralymphatically, parenterally, subcutaneously, intramuscularly, intradermally, intraperitoneally, interstitially, intraarticulary, intranasaly, orally, or topically using a variety of dosage forms. For example, ultrasound contrast agents are administered by intravenous injection or by injection into the urinary bladder.

In particular, in form of a pharmaceutical composition for microdosing enables to reach vascular targets, like endothelium or macrophages in vessels, but also areas in the tissue having an altered permeability, like tumors or a side of inflammation, but also intestinal targets.

Using microdosing of functionalised ubbles or functionalised particles in form of a pharmaceutical or diagnostic composition according to the present invention allows e.g. for characterising angiogenesis and inflammation in tumors, atherosclerosis and rheumatoid diseases. Further, the use of microdosing is particularly useful in all interventional applications, e.g. interventional coronary angiography. It is possible to differentiate plugs etc. Even repeated diagnosis is possible since the whole body dosage of the repeated microdosing is below the amount of ordinary high dosage diagnosis or treatment.

The pharmaceutical or diagnostic compositions according to the present invention may be used in combination with known diagnostic methods, like optical imaging, MRI, CT, PET or SPECT.

In the following, the invention will be described in more detail by reference to examples. The examples are provided for illustrating the claimed matter without limiting the same.

### Example 1

Air-filled polybutylcyanoacrylate microparticles (PBCA-MPs) were prepared following the one-step-method described in Hauff P. et.al., Radiology 2004, 231:667-673. Alternatively, PBCA-MPs were prepared according to the two-step-method described in Schmidt & Rössling, Chemical Engineering Science, 2006, 4973-4981. The microparticles prepared were stored at concentrations of 2.2x10⁹ microparticles/ml.

### Example 2

Preparation of the aqueous liquid storage medium allowing stabilisation of PBCA-MP in concentrations below 5x10⁸ MP/ml.

For preparing the aqueous liquid storage medium, PBCA-MPs were prepared as described for the one-step-method identified in the above. In brief, 300ml 1% Triton X-100 solution (pH 2.5), 3g BCA-monomer were dispersed using an Ultra Turrax T50 disolver for 90 minutes on highest level (IKA, Germany). The suspension were placed in a separation funnel and floated therein for 90 hours. Thereafter, the subnatant is separated and diluted 1:100 with water and stirred overnight with a magnetic stirrer. The suspension obtained now containing 0.01% Triton X-100 were allowed to stand for at least 24 hours at room temperature to obtain the aqueous liquid storage medium according to the present invention. Finally, said medium was filtered sterile (0.2 µm, Stericup Millipore).

### Example 3

Determining stability of PBC-MP obtained in example 1 in a medium prepared according to example 2 and compared to other media.

For testing the effect of different media on the stability of microparticles obtained in example 1, low concentrations of said microparticles were suspended in the aqueous liquid storage medium obtained in example 2 and compared with suspensions in relevant standard media (A: Ultrapurewater; B: Millipore water plus surfactant (0.01% Trition X-100) (media A and B represent the state of the art media used for storage of microparticles)). The stock solution of microparticles having a concentration of 2.2x10⁹ MP/ml were diluted in the three different media as follows: 1:2,000 corresponding to 1.1x10⁶ MP/ml, 1:4,000 corresponding to 5.5x10⁵ MP/ml and 1:8,000 corresponding to 2.7x10⁵ MP/ml. The different suspensions containing the diluted microparticles are allowed to stand for various time points (15 minutes, 60 minutes and 120 minutes) and analysed thereafter. A characterisation of the PBCA-MP stored for the different time points in the different storage media were done by flow cytometry analysis using a FACS calibur (Becton Dickinson, USA), based on forward and sideward scatter vaules. The results are shown in figures 1 (15 minutes), 2 (60 minutes) and 3 (120 minutes), respectively.

As shown in figure 1, after 15 minutes incubation, aggregates are formed in water without surfactant at different concentrations while in the storage medium according to the present invention, the microparticles are stable. In the medium containing 0.01% Triton X-100, an increasing instability and formation of aggregates can be observed for higher dilutions.

After 60 minutes incubation, decomposition and degradation can be observed in the water media. The same is true for the water including the lower amount of surfactant. In contrast, in the storage medium according to the present invention, the microparticles are stable independently of the dilution.

In figure 3, the results after 120 minutes are shown. In the medium containing pure water the particles are decomposed and fragments are determined. The same is true for the microparticles stored in water including 0.01% surfactant. In contrast, the microparticles stored in the storage medium according to the present invention remain stable particularly when compared with the starting solution.

To conclude, as demonstrated herein, the suspension of non-functionalised or functionalised bubbles or non-functionalised or functionalised particles in an aqueous liquid medium as well as the aqueous liquid storage medium according to the present invention have superior properties regarding storage stability in particular with respect to concentrations of said bubbles or particles compared to state of the art media. The suspension and aqueous storage liquid media are particularly useful for storing the bubbles or particles over a longer period of time having good storage stability, allowing to permit manufacture and a central location and distribution to a storage at hospitals etc. prior to use. In particular, the suspension and aqueous liquid storage medium according to the present invention allow providing ready-to-use compositions, in particular, ready-to-use compositions for microdosing of the bubbles or particles. Furthermore, the suspension and aqueous liquid storage medium according to the present invention allows manipulating and characterizing of said bubbles or particles even in very low concentrations.

## Claims

1. A suspension of non-functionalised or functionalised micro- or nanobubbles or non-functionalised or functionalised micro- or nanoparticles in an aqueous liquid medium, usable for diagnostic, therapeutic and analytic purposes **characterised in that** the liquid medium contains dissolved shell material of said micro- or nanobubbles or micro- or nanoparticles in a concentration avoiding the dissolution or the loss of acoustic properties or other properties of said micro- or nanobubbles or micro- or nanoparticles.

2. The suspension of bubbles or particles in an aqueous liquid medium according to claim 1 wherein the concentration of said bubbles or particles is below 1x10¹⁰/ml, preferably below 1x10⁹/ml, e.g. wherein the concentration of said bubbles or particles is below 5x10⁸/ml, preferably below 1x10⁸/ml.

3. The suspension of bubbles or particles in an aqueous liquid medium according to any one of the preceding claims wherein the concentration of said bubbles or particles is below 5x10⁷ml, preferably below 5x10⁶ bubbles or particles/ml, in particular, the concentration is below 5x10⁵ bubbles or particles/ml, more preferably 1x10⁵ bubbles or particles/ml.

4. The suspension of bubbles or particles in an aqueous liquid medium according to any one of the preceding claims wherein the total concentration of the shell forming material of said particles or bubbles present in the bubbles or particles as well as dissolved components is adapted allowing to administer the suspension in a concentration of below 143 µg/kg body weight, preferably below 14,3 µg/kg body weight., in particular wherein the total concentration of the shell forming material is below 1,43 µg/kg body weight.

5. The suspension of bubbles or particles in an aqueous liquid medium according to any one of the preceding claims further containing a surfactant, preferably, a non-ionic surfactant.

6. The suspension of bubbles or particles in an aqueous liquid medium according to any one of the preceding claims wherein the dissolved components of the shell material consist of the polymer and/or monomer identical with the polymer and/or monomer forming the bubble or the particle.

7. The suspension of bubbles or particles in an aqueous liquid medium according to any one of the preceding claims wherein said bubbles or particles comprises polyalkylcyanoacrylates, preferably functionalised polyalkylcyanoacrylates; homopolymers, in particular, polyethylene, polypropylene, polystyrole, polyvinylchloride and/or polyamide; copolymers, in particular, polyester, polyurethane and/or polyamide; neutral biopolymers, in particular, polysaccarides, proteins and peptides, and/or polynucleotides; biopolymers, in particular, polylactids or polyhydroxybutyrats; and/or synthetic biopolymers, in particular, polyesters, polyvinyl-alcohols, polybutylenadipat-terephthalat, polybutylensuccinat, polycaprolactone, polyglycolids, in particular, polyalkylcyanoacrylates, preferably functionalised polybutylcyanoacrylates.

8. The suspension of bubbles or particles in an aqueous liquid medium according to any one of the preceding claims in form of a ready-to-use solution.

9. A method for preparing a suspension of non-functionalised or functionalised micro- or nanobubbles or non-functionalised or functionalised micro-or nanoparticles in an aqueous liquid medium having improved shelf life comprising the step of
a) providing micro- or nanobubbles and/or micro- or nanoparticles,
b) providing an aqueous liquid medium containing dissolved shell material of the micro- or nanobubbles or micro- or nanoparticles according to step a) in a concentration avoiding the dissolution or the loss of acoustic properties or other properties of said micro- or nanobubbles or micro- or nanoparticles.
c) suspending the micro- or nanobubbles and/or micro- or nanoparticles of step a) in the aqueous liquid medium of step b) to provide a suspension of non-functionalised or functionalised micro- or nanobubbles or non-functionalised or functionalised micro- or nanoparticles in an aqueous liquid medium having improved shelf life.

10. A method for preparing a suspension of non-functionalised or functionalised micro- or nanobubbles or non-functionalised or functionalised micro-or nanoparticles in an aqueous liquid medium having improved shelf life according to claim 9 comprising the steps of
a) preparing micro- or nanoparticles and/or micro- or nanobubbles in a reaction medium containing monomers and, optionally, a surfactant;
b) separating the micro- or nanobubbles or micro- or nanoparticles obtained in step a), e. g. by floating;
c) optionally diluting the remaining reaction medium with an aqueous solution, preferably water, and stirring said reaction medium;
d) allowing the reaction medium to stay for a predetermined time for obtaining a reaction medium containing monomeric or polymeric components.
e) mixing micro- or nanobubbles or micro- or nanoparticles, e.g. obtained in step b), with the reaction medium obtained in step d) to obtain a suspension of micro- or nanobubbles or micro- or nanoparticles in an aqueous liquid medium.

11. The method according to claim 9 or 10 wherein the reaction medium is a medium being saturated with the monomeric or polymeric components.

12. The method according to any one of claims 9 to 11 wherein the surfactant is a non-ionic surfactant, preferably the surfactant is present in an amount of below 0.05% in the suspension obtained.

13. Aqueous liquid storage solution for micro- or nanobubbles or micro- or nanoparticles, optionally comprising a surfactant, **characterised in that** the medium contains dissolved components of the shell material of said micro- or nanobubbles or micro- or nanoparticles to be stored therein in a concentration avoiding the dissolution or the loss of acoustic properties or other properties of the mentioned micro- or nanobubbles or micro- or nanoparticles.

14. Pharmaceutical composition having extended shelf life containing non-functionalised or functionalised micro- or nanobubbles or non-functionalised or functionalised micro- or nanoparticles in an aqueous liquid medium for diagnostic and therapeutic purposes in a suspension as defined in any one of claims 1 to 7.

15. The pharmaceutical composition according to claim 14 wherein the non-functionalized or functionalized microbubbles or non-functionalized or functionalized microparticles are present in a concentration of below 5 x 10⁸ microbubbles or microparticles per ml, preferably 5 x 10⁷, like 5 x 10⁶, in particular, below 5 x 10⁵.

16. The pharmaceutical composition according to 14 or 15 in form of a ready-to-use solution or in form a re-suspension having an extended shelf life.

17. The suspension according to any one of claims 1 to 8 for use in therapy and diagnosis, in particular, for use in diagnostic methods including in vivo imaging, preferably, PET or SPECT.
